# EUROPEAN PATENT APPLICATION

(11) **EP 0 619 105 A1**
(43) Date of publication of application: **12.10.1994**
(21) Application number: 94302409.1
(22) Date of filing: 05.04.1994
(51) Int. Cl.: A61F 13/02

(54) **Surgical wound dressings**

(30) Priority: 07.04.1993 GB 9307312
(71) Applicant: UNITED SURGICAL SERVICES LTD., Worcester WR1 3DG (GB)
(72) Inventor: Strover, Angus Everett, Worcester WR1 3DG (GB)
(74) Representative: Hulse, Thomas Arnold

(57) **Abstract**

A "patient-friendly" surgical wound dressing (10) comprises an absorbent layer (11) having an area commensurate with the extent of the wound (12) to be dressed, an occlusive layer (13) overlying and extending beyond the edges (14) of the absorbent layer, a suction tube (15) between the absorbent layer (11) and the occlusive layer (13) and open to the absorbent layer (through holes 16), self-adhesive at least along marginal portions (17) of the side of the occlusive layer facing the absorbent layer, and at least one free end (18) of the suction tube (15) projecting out from the occlusive layer for ready connection to suction apparatus

## Description

This invention relates to surgical wound dressings.

Absorbent dressings, occlusive dressings and suction drainage are well known in the treatment of surgical wounds.

Absorbent dressings. These absorb and retain blood and serum from the vicinity of the wound. These dressings are made of a variety of natural and synthetic fabrics (such as cotton, and polyesters) and are applied to wounds to collect blood and exudate coming out of the wound. They are usually changed and discarded in the interests of the comfort and hygiene of the patient. Absorbent dressings are sterile or surgically clean and facilitate the healing of the wound by preventing contamination or the introduction of pathogenic bacteria from without.

Absorbent dressings suffer from the disadvantages of sometimes becoming heavily impregnated with blood and serum. This may lead to maceration of the skin surrounding the wound with delay in wound healing. The blood or wound exudate may leak through the dressing and soil the clothes and bedclothes of the patient. Drying of the blood and exudate in a fabric dressing may create a hard composite with the fabric of the dressing which then becomes uncomfortable for the patient. In cases where circumferential bandages have been used on the limbs, the stiff or hardened dressing can create a danger to the venous circulation of the limb with complications such as swelling, venous occlusion, venous thrombosis and circulatory occlusion of the limb.

When wet or heavily impregnated with wound exudate, fabric dressings may allow the growth of pathological organisms within the dressing. This constitutes a danger of wound infection.

Occlusive dressings. These are made of membranes which occlude the passage of blood and fluids. They can protect the clothes and bedclothes from wound leakage and conversely, they serve to protect the wound from possible contamination during bathing. Occlusive dressings can also be used in cases, such as burns or trauma, involving massive skin loss, to prevent excessive drying of the wound with subsequent dehydration of the patient. Occlusive dressings are usually adhesive and there are several types at present on the market, such as OPSITE sold by Smith and Nephew Ltd., and TAGADERM sold by 3Ms Medical.

When used by themselves early in the management of surgical wounds, occlusive dressings suffer from the disadvantage of accumulation of excessive fluid beneath the dressing which may leak out of the edges. They are therefore often used in combination with fabric dressings under these circumstances.

If pathogenic bacteria become trapped under occlusive dressings there is a danger of proliferation thus infecting the wound.

Suction drainage. After surgical closure of the wounds, drains in the form of perforated plastic pipes connected to a suction apparatus are commonly left in the superficial or deep layers of the wound with the purpose of preventing haematoma, which is excessive accumulation of blood in the wound.

By aspirating the excessive blood from the wound, suction drainage facilitates healing of the tissues by reducing the physical space between the cut surfaces of the wound. Furthermore, the volume of fluid aspirated into the suction apparatus can be collected and measured which allows the medical staff to estimate accurately the loss of blood from the patient.

Suction drains reduce the amount of exudate or blood welling out of the wound into fabric dressings, thereby reducing the frequency with which fabric dressings need to be changed. They also have similar advantages when used in the tissues under occlusive dressings.

Suction drains when left in orthopaedic wounds close to cancellous or spongy bone can occasionally aspirate excessive blood from the bone and constitute a danger to the patient. They are also uncomfortable in the wound and painful when removed after one or more days. Suction drains also theoretically constitute a vehicle for the invasion of bacteria into the wound. In general, however, their advantages outweigh their disadvantages and they are extensively used in surgery.

The object of the present invention is to obviate the disadvantages of the above while maximising on their advantages.

According to the present invention, a surgical wound dressing comprises an absorbent layer having an area commensurate with the extent of the wound to be dressed, an occlusive layer overlying and extending beyond the edges of the absorbent layer, a suction tube between the absorbent layer and the occlusive layer and open to the absorbent layer, self-adhesive at least along marginal portions of the side of the occlusive layer facing the aborbent layer, and at least one free end of the suction tube projecting out from the occlusive layer for ready connection to suction apparatus.

The self-adhesive enables the occlusive layer to be adhered to the skin clear of the wound to which the absorbent layer is applied, the latter collects the blood or exudate from the surface of the wound by capillary action, and suction apparatus connected to the suction tube aspirates and collects excessive blood and exudate from within the dressing so that this fluid can be measured. It will be evident that the dressing of the present invention combines the advantages of absorbent dressings, occlusive dressings and suction drains, per se, while obviating the disadvantages of all three, in that the wound is kept dry and hygienic as no fluid is allowed to collect in the dressing apart from the very small amount which saturates the absorbent layer before suction can be effective, the bedclothing is protected by the occlusive layer from being soiled by blood and exudate, the self-adhesive on the occlusive layer obviates the need for circumferential bandages with their accompanying danger to the vascular circulation of limbs, and discomfort or pain of inserted drains and pain when removed is avoided, plus the danger of deep and superficial infection.

Conveniently, the self-adhesive covers the whole of the occlusive layer so that it serves to hold the suction tube and the absorbent layer thereto during manufacture, storage and transport, and application of the dressing; and it will be evident that a releasable backing of at least equal extent to the occlusive layer will preferably be applied during manufacture to protect the adhesive and the absorbent layer. Again, it will be evident that the dressing will be sealed after manufacture in a sterile package.

The suction tube may be a looped tube with holes in the wall thereof spaced along the loop and facing the absorbent layer; and a plastics collar or connector may be moulded on to the tube and may be sealed to the occlusive dressing at the time of manufacture.

Once the aspiration of blood and/or exudate from a wound has ceased (which usually occurs in the first twenty-four hours postoperatively) the suction tube need not be removed from the dressing but can simply be cut off and the open end (or ends) sealed by applying a small adhesive dressing.

Embodiments of the invention will now be described, by way of example only, with reference to the accompanying drawings, in which:-
Figure 1 is a diagrammatic fragmentary perspective view, partially broken away, showing the construction and application of a surgical wound dressing in accordance with the invention;
Figure 2 is an underneath plan view showing an initial stage in the manufacture of a surgical wound dressing in accordance with the invention;
Figure 3 corresponds to Figure 2, but on a smaller scale showing further stages in the manufacture of the dressing; and
Figures 4 and 5 correspond to Figure 3 but show stages in preparing and completing the dressing for storing and transporting.

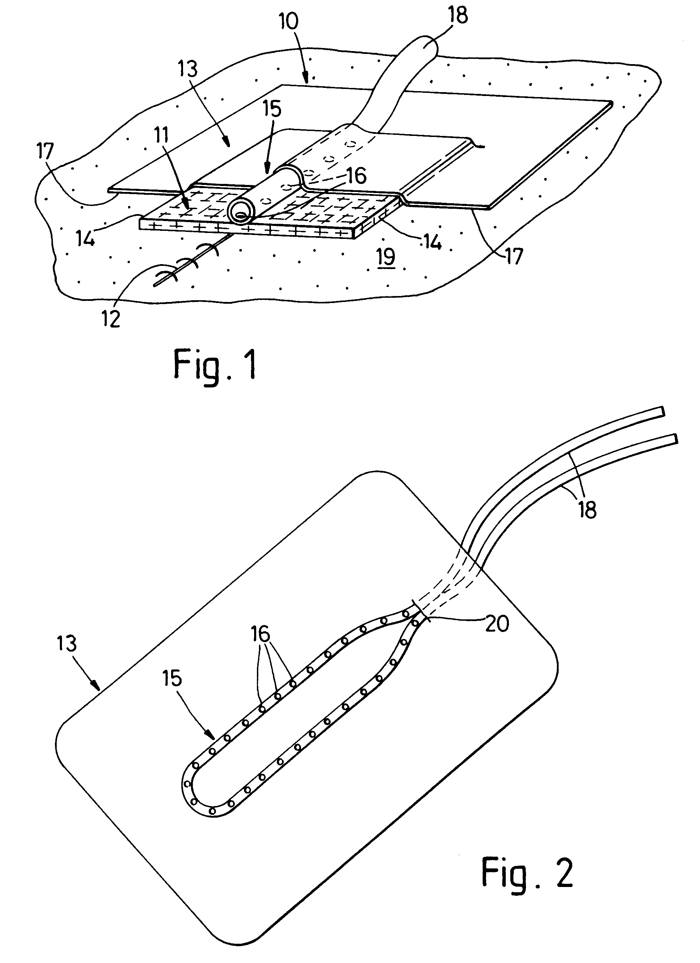

In Figure 1, a surgical wound dressing 10 comprises an absorbent layer 11 having an area commensurate with the extent of the wound 12 to be dressed, an occlusive layer 13 overlying and extending beyond the edges 14 of the absorbent layer, a suction tube 15 between the absorbent layer 11 and the occlusive layer 13 and open to the absorbent layer (through holes 16), self-adhesive at least along marginal portions 17 of the side of the occlusive layer facing the absorbent layer, and at least one free end 18 of the suction tube 15 projecting out from the occlusive layer for ready connection to suction apparatus (not shown).

The occlusive layer 13 can be seen to be adhered to the skin 19 clear of the wound 12 to which the absorbent layer 11 is applied. The latter collects the blood or exudate from the surface of the wound by capillary action, and the suction apparatus (not shown) connected to the suction tube 15 aspirates and collects excessive blood and exudate from within the dressing and drawn through the holes 16 in the suction tube so that this fluid can be measured.

The dressing combines the advantages of absorbent dressings, occlusive dressings and suction drains, per se, while obviating the disadvantages of all three in the manner described above.

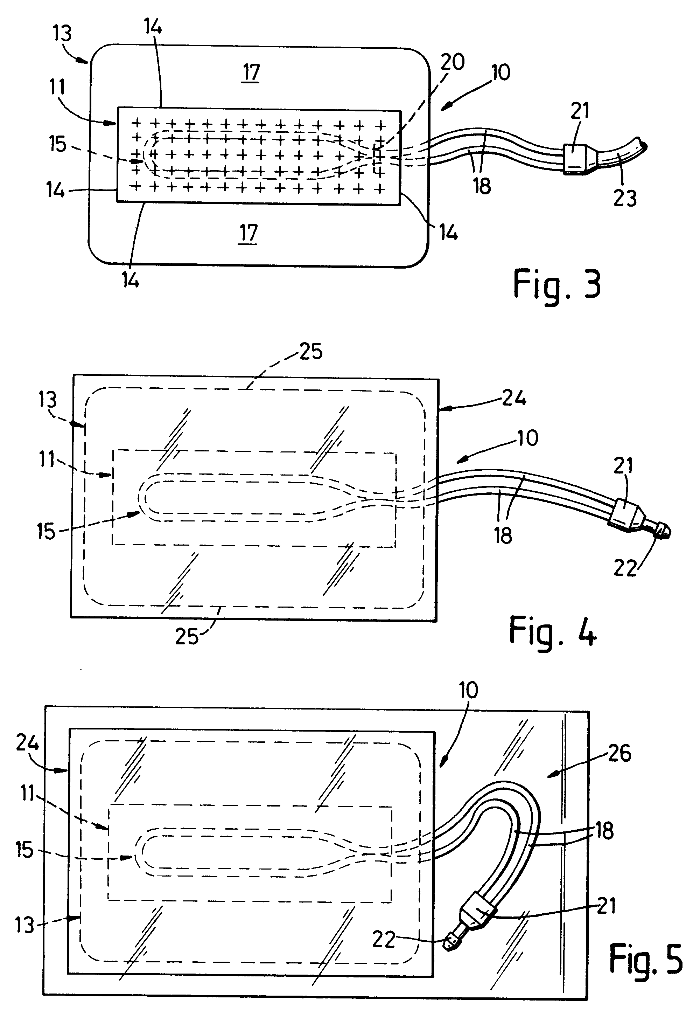

Conveniently, the self-adhesive covers the whole of the occlusive layer 13 so that it serves to hold the suction tube 15 (as shown in Figure 2) and the absorbent layer 11 (as shown in Figure 3) thereto during manufacture, storage and transport (see also Figures 4 and 5), as well as during application of the dressing.

In Figure 2 the suction tube 15 is a looped tube with holes 16 spaced along the loop and is applied to the all-over self-adhesive coated side of the occlusive layer 13 so that the holes face the absorbent layer 11 subsequently applied as shown in Figure 3 to leave the exposed self-adhesive along the marginal portions 17 of the occlusive layer.

The non-perforated free end lengths 18 of the suction tube pass through a slit 20 in the occlusive layer 13 and extend to a connector 21 moulded thereon (Figures 3 to 5) and having a nipple 22 (or other means) for attachment of a pipe 23 (see Figure 3) for coupling to the suction apparatus (not shown).

In Figure 4, a releasable backing 24 is shown covering and extending beyond the edges 25 of the occlusive layer 13 to protect the adhesive and the absorbent layer 11, while in

Figure 5 the dressing 10 is shown sealed in a transparent sterile package 26.

## Claims

1. A surgical wound dressing (10) characterised by an absorbent layer (11) having an area commensurate with the extent of the wound (12) to be dressed, an occlusive layer (13) overlying and extending beyond the edges (14) of the absorbent layer, a suction tube (15) between the absorbent layer and the occlusive layer and open to the absorbent layer, self-adhesive at least along marginal portions (17) of the sides of the occlusive layer facing the aborbent layer, and at least one free end (18) of the suction tube (15) projecting out from the occlusive layer (13) for ready connection to suction apparatus.

2. A dressing as in Claim 1, characterised in that the self-adhesive covers the whole of the occlusive layer (13) so that it serves to hold the suction tube (15) and the absorbent layer (11) thereto during manufacture, storage and transport, and application of the dressing.

3. A dressing as in Claim 1 or Claim 2, characterised in that a releasable backing (24) of at least equal extent to the occlusive layer (13) is applied during manufacture to protect the adhesive and the absorbent layer (11).

4. A dressing as in Claim 3, characterised in that after manufacture it is sealed in a sterile package (26).

5. A dressing as in any one of Claims 1 to 4, characterised in that the suction tube (15) is a looped tube with holes (16) in the wall thereof spaced along the loop and facing the absorbent layer (11).

6. A dressing as in Claim 5, characterised in that a plastics collar or connector (21) is moulded on to the suction tube (15).

7. A dressing as in Claim 6, characterised in that the plastics collar or connector (21) is sealed to the occlusive dressing (13) at the time of manufacture.
